# EUROPÄISCHE PATENTSCHRIFT

(11) **EP 4 181 672 B1**
(45) Veröffentlichungstag und Bekanntmachung des Hinweises auf die Patenterteilung: **10.04.2024**
(21) Anmeldenummer: 22786366.9
(22) Anmeldetag: 23.09.2022
(51) Int. Cl.: A01K 67/033

(54) **VERFAHREN ZUR BEREITSTELLUNG VON FUTTERMITTELN IN EINER ANLAGE ZUR AUFZUCHT VON INSEKTENLARVEN**
METHOD FOR PROVIDING FEED IN AN INSTALLATION FOR REARING INSECT LARVAE
PROCÉDÉ DE PRÉPARATION D'ALIMENTS POUR ANIMAUX DANS UNE INSTALLATION D'ÉLEVAGE DE LARVES D'INSECTES

(30) Priorität: 08.10.2021 DE 102021005045; 15.10.2021 DE 102021005045
(43) Veröffentlichungstag der Anmeldung: 24.05.2023
(73) Patentinhaber: WEDA-Dammann & Westerkamp GmbH, 49424 Goldenstedt (DE)
(72) Erfinder: WESTERKAMP, Klemens, 49424 Goldenstedt (DE)
(74) Vertreter: Weeg, Thomas
(86) Internationale Anmeldenummer: PCT/EP2022/076561
(87) Internationale Veröffentlichungsnummer: WO 2023/057240

(56) Entgegenhaltungen:
- EP-A1- 2 986 107
- EP-A2- 2 153 716

## Beschreibung

Die vorliegende Erfindung bezieht sich auf ein Verfahren zur Bereitstellung von Futtermitteln nach dem Oberbegriff des Anspruchs 1. Die Erfindung bezieht sich auch auf eine Anlage mit einer Vorrichtung zur Einbringung eines Futterbreis in einen Behälter nach dem Oberbegriff des Anspruchs 10.

Aus den Schriften DE 10 2020 005 146 A1 und EP 2 986 107 A1 ist das gattungsgemäße Verfahren bekannt, in einer Anlage zur Aufzucht von Insektenlarven einen Futterbrei in einen Zuchtbehälter zu geben. Danach werden Insektenlarven auf den Futterbrei gesetzt. Der Zuchtbehälter wird dann mit dem Futterbrei und darauf platzierten Insektenlarven über einen Zeitraum hinweg in einer Wärmekammer gelagert, damit sich die Insektenlarven entwickeln können.

Wenn der Futterbrei in den Zuchtbehälter eingefüllt wird, muss er ausreichend flüssig sein, um sich darin gut verteilen zu können. Auch ist eine noch fließfähige Konsistenz des Futterbreis erforderlich, damit es in den Förderanlagen für den Futterbrei noch pumpfähig ist. Aus der Schrift EP 2 153 716 A2 ist eine für den Anspruch 10 gattungsbildende Anlage bekannt, schnell und bedarfsgenau eine angeforderte Futtermenge bereitzustellen. Bei einer unzureichenden Verteilung des Futterbreis über die Grundfläche des Zuchtbehälters wird das volle Aufzuchtpotenzial des Zuchtbehälters nicht vollständig ausgenutzt. Bei dem bekannten Futterbrei wird aus diesen Gründen ein Trockensubstanzgehalt von bis zu 35% angestrebt, sodass der in den Zuchtbehälter gefüllte Futterbrei einen Flüssigkeitsanteil von zumindest etwa 65 % aufweist. Andererseits ist es wünschenswert, zum Ende der Brutphase hin einen möglichst eingedickten oder sogar angetrockneten Futterrest vom Futterbrei zu haben, damit die Larven nach der Entnahme des Zuchtbehälters aus der Wärmekammer möglichst einfach aus dem Zuchtbehälter ausgekippt und von den Futterresten getrennt werden können. Die Eindickung des Futterbreis erfolgt über eine Verdunstung der Flüssigkeitsanteile aus dem Futterbrei in der Wärmekammer und die Abfuhr der mit der verdunsteten Flüssigkeit angereicherten Luft aus der Wärmekammer. Durch die Verdunstung und Entlüftung entsteht ein hoher Energieverbrauch, der aus Effizienzgründen möglichst niedrig gehalten werden sollte. Hat der Futterbrei beim Einfüllen in den Zuchtbehälter einen geringen Flüssigkeitsanteil, um den Energieverbrauch zu begrenzen, ist der Futterbrei aber zu pastös, um sich dort gut verteilen zu können.

Es ist die Aufgabe der vorliegenden Erfindung, das Verfahren zu verbessern, indem im Zuchtbehälter ein Futterbrei ausgebracht wird, der gut pumpfähig ist und sich gut im Zuchtbehälter verteilt, der aber einen möglichst hohen Trockenmasseanteil aufweist.

Die Aufgabe wird für ein gattungsgemäßes Verfahren mit den kennzeichnenden Merkmalen des Anspruchs 1 gelöst.

Die Aufgabe wird für eine gattungsgemäße Anlage mit den kennzeichnenden Merkmalen des Anspruchs 10 gelöst.

Durch die voneinander getrennte Einbringung von Futterfraktionen mit unterschiedlichen Flüssigkeitsanteilen ist es möglich, eine erste Futterfraktion in den Zuchtbehälter einzubringen, die einen höheren Flüssigkeitsanteil aufweist und sich dadurch sehr gut in dem Zuchtbehälter verteilt. Die zweite Futterfraktion weist dann einen niedrigeren Flüssigkeitsanteil auf. Wenn die zweite Futterfraktion über dessen Grundfläche verteilt in den Zuchtbehälter aufgebracht wird, ergibt sich auch für die zweite Futterfraktion über das Aufbringen eine zumindest annähernd gleichmäßige Verteilung. Nach dem Aufbringen kann die zweite Futterfraktion Flüssigkeit aus der ersten Futterfraktion in sich aufsaugen, sodass sich die Flüssigkeitsverteilung zwischen den beiden Futterfraktionen zumindest annähernd ausgleicht. Wenn dabei schon die erste Futterfraktion einen vergleichsweise niedrigen Flüssigkeitsanteil aufweist, wird der gesamte Flüssigkeitsanteil in beiden Futterfraktionen zusammen auf einen Wert verringert, bei dem sich ein einstufig eingebrachter Futterbrei nicht mehr ausreichend innerhalb des Zuchtbehälters verteilt hätte. Gleichwohl ergibt sich durch die voneinander getrennte Einbringung der Futterfraktionen eine gute Verteilung des Futterbreis in dem Zuchtbehälter.

Durch die zweite Futterfraktion mit einem niedrigeren Flüssigkeitsanteil und die Angleichung der Flüssigkeitsanteile in den verschiedenen Futterfraktionen auf einen gemeinsamen Durchschnittswert nach dem Einfüllen beider Futterfraktionen in den Zuchtbehälter wird für den gesamten in den Zuchtbehälter eingebrachten Futterbrei ein Flüssigkeitsanteil erreicht, der niedriger ist als in einem pumpfähigen Futterbrei, der in einem einzigen Verfahrensschritt in den Zuchtbehälter eingefüllt wird. Durch den vergleichsweise niedrigen Flüssigkeitsanteil in dem Futterbrei aus der ersten und zweiten Futterfraktion ist der Energie- und Lüftungsaufwand zum Heruntertrocknen des Futterbreis in der Wärmekammer erheblich niedriger als bei der herkömmlichen Befüllung des Zuchtbehälters.

Die erste Futterfraktion kann einen Feuchtegehalt aufweisen, bei dem diese immer noch gut pumpfähig ist. Das ist beispielsweise bei einem Feuchtegehalt von mindestens 65 % noch möglich. Die Anmischung, Verteilung und Dosierung dieser Futterfraktion kann dadurch mit einfachen, aus der allgemeinen auch für andere Nutztiere bekannten Fütterungstechnik mit bekannten und bewährten Komponenten erfolgen. Die Reinhaltung und Wartung der Komponenten für die Förderung und Ausdosierung der ersten Futterfraktion ist auf einfache Weise möglich.

Die voneinander getrennte Einbringung der Futterfraktionen bedeutet nicht zwangsläufig, dass diese zeitlich nacheinander in den Zuchtbehälter eingebracht werden müssen. Das kann vielmehr auch ganz oder teilweise zeitgleich erfolgen. Bei einer zeitlich aufeinanderfolgenden Einbringung an räumlich voneinander getrennten Stationen einer Dosieranlage können die für die Einbringung verwendeten Maschinenkomponenten allerdings einfacher auf ihre jeweilige Verteil- und Einbringfunktion hin ausgelegt werden. Der Zuchtbehälter kann dafür beispielsweise auf ein Förderband gestellt werden, dass den Zuchtbehälter an den Stationen für die Zudosierung der ersten Futterfraktion, der zweiten Futterfraktion und der Insektenlarven entlang fördert.

Die Mischbehälter können dazu vorgesehen sein, darin ein Flüssigfutter oder ein Trockenfutter anzumischen. Bei dem Mischbehälter kann es sich aber auch um einen reinen Lagerbehälter handeln, in den ein fertig gemischtes Flüssig- oder Trockenfutter oder einer Trockenfutterkomponente eingelagert wird und aus dem die jeweiligen Futterportionen bei Bedarf in einer entsprechenden Menge entnommen werden. Bei einem Vorratsbehälter für Trockenfutter kann es sich beispielsweise um ein oder mehrere Silos für Trockenfutter und/oder Trockenkomponenten handeln, die erst noch zu einer fertigen Mischung eines Trockenfutters verarbeitet werden müssen. Trockene Futterkomponenten können auch als Futterfraktionen für Flüssigfutter in einem oder mehreren Silos vorgehalten werden, um diese später mit Wasser oder anderen Flüssigkeiten zu einem Flüssigfutter zusammenzumischen. Tanks können als Vorratsbehälter für Flüssigfutter dienen.

Nach einer Ausgestaltung der Erfindung handelt es sich bei der zweiten Futterfraktion um ein Trockenfutter oder eine Trockenfutterkomponente. Wenn hier von Trockenfutter oder einer Trockenfutterkomponente die Rede ist, so ist damit ein Futter gemeint, das eine Trockensubstanz von mindestens 65 % der Gesamtmasse der ausgegebenen zweiten Futterfraktion aufweist. Ein solches Trockenfutter oder eine solche Trockenfutterkomponente hat den Vorteil, dass es mit den bekannten Trockenfutterverteilanlagen und der im Rohrleitungsnetz befindlichen Fördertechnik störungsfrei und ohne einen zusätzlichen erheblichen Reinigungsaufwand förderbar ist. Dieses Trockenfutter oder diese Trockenfutterkomponente können aus verschiedenen Futterkomponenten zusammengesetzt sein. Ein Trockenfutter ist heute häufig ein Mischfutter, das aus mindestens zwei, aber auch zehn oder mehr Einzelfuttermitteln zusammengesetzt sein kann, dem zusätzlich noch Futtermittelzusatzstoffe wie beispielsweise Vitamine, Aminosäuren, Spurenelemente oder Enzyme beigemischt sein können. Auch Fermente wie beispielsweise Milchsäurebakterien können zugesetzt sein. Außerdem können noch technologische Zusatzstoffe wie Konservierungsmittel, Bindemittel, Emulgatoren, Antioxidationsmittel oder Silierzusatzstoffe zugesetzt sein.

Nach einer Ausgestaltung der Erfindung wird das Trockenfutter oder die Trockenfutterkomponente vor der Einbringung in den Zuchtbehälter in einem Mischbehälter aus verschiedenen Futterkomponenten zusammengemischt, wobei die verwendeten Futterkomponenten aus zu der Anlage gehörigen Vorratsbehältern entnommen und dem Mischbehälter zugefördert werden. Als eine Futterkomponente ist dabei auch der Zusatz von Wasser zu sehen, soweit im Rahmen einer Rezeptur der Zusatz von Wasser zu einer Charge des Trockenfutters vorgesehen ist. Aus den verschiedenen Futterkomponenten kann eine Futtercharge individuell zusammen gemischt und in den Zuchtbehälter befördert werden. Die einzelnen Futterkomponenten werden bevorzugt in einem Mischer miteinander verrührt, bevor die fertige Mischung in den Zuchtbehälter abgegeben wird.

Nach einer Ausgestaltung der Erfindung wird das Trockenfutter oder eine Trockenfutterkomponente dem jeweiligen Zuchtbehälter aus einem Klappkasten, einer Vorrichtung zur Volumen- oder Zeitdosierung oder einer Zellenradschleuse oder einer anderen Verteil- und Zudosiervorrichtung zugeführt. Der Klappkasten hat sich als Abgabemittel für Trockenfutter oder eine Trockenfutterkomponente bewährt. Im Klappkasten kann eine gewünschte Menge einer Trockenfuttercharge angesammelt und durch Öffnen der bodenseitigen Klappen an einen Zuchtbehälter abgegeben werden. Der Klappkasten kann in einem automatisierten Betrieb genutzt werden, sodass manuelle Hilfsarbeiten bei der Befüllung des Klappkastens mit einer Trockenfuttercharge und deren Abgabe an den Zuchtbehälter nicht erforderlich sind. Aber auch andere Fördertechniken wie beispielsweise Schneckenförderer oder andere Förder- und Dosiermittel kommen als Zuführmittel in Betracht.

Nach einer Ausgestaltung der Erfindung wird eine Portion eines Trockenfutters oder einer Trockenfutterkomponente vor der Zuführung in den Zuchtbehälter verwogen oder die Abgabemenge mittels einer Vorrichtung zur Durchlaufmengenmessung mengendosiert. Durch ein Verwiegen des Mischbehälters oder des Klappkastens mit einer Waage oder die Bestimmung der geförderten Menge des Trockenfutters oder der Trockenfutterkomponente mit einer sonstigen Vorrichtung zur Durchlaufmengenmessung wie beispielsweise einer Durchlaufwaage kann eine vorbestimmte Portionsmenge einer Trockenfuttercharge genau eingestellt, kontrolliert und dokumentiert werden. Eine Trockenfuttercharge kann auf diese Weise sehr gut automatisiert nach einem vorgegebenen Rezept in einer passenden Menge hergestellt und in den Zuchtbehälter ausgegeben werden.

Nach einer Ausgestaltung der Erfindung wird eine Portion eines Flüssigfutters vor der Zuführung in den Zuchtbehälter verwogen oder die Abgabemenge mittels einer Vorrichtung zur Durchflussmengenmessung mengendosiert. Durch ein Verwiegen des Mischbehälters oder einer Förderleitung mit einer Waage oder die Bestimmung der geförderten Menge des Flüssigfutters kann eine vorbestimmte Portionsmenge einer Flüssigfuttercharge genau eingestellt, kontrolliert und dokumentiert werden. Eine Flüssigfuttercharge kann auf diese Weise sehr gut automatisiert nach einem vorgegebenen Rezept in einer passenden Menge hergestellt und in den Zuchtbehälter ausgegeben werden.

Nach einer Ausgestaltung der Erfindung werden zuerst die erste Futterfraktion und danach die zweite Futterfraktion in den Zuchtbehälter eingegeben. Bei dieser Reihenfolge der Zugabe der Futterfraktionen in den Zuchtbehälter kann sich die Futterfraktion mit dem höheren Feuchteanteil zunächst gleichmäßig in dem Zuchtbehälter verteilen, bevor dann die zweite Futterfraktion hinzu gegeben wird. Durch das Aufbringen der zweiten Futterfraktion auf die erste Futterfraktion ergibt sich eine Mischung der beiden Fraktionen als ein Futterbrei, auf den danach die Insektenlarven gut aufgebracht werden können.

Nach einer Ausgestaltung der Erfindung wird mit einem Feuchtigkeitssensor der Feuchteanteil in der ersten und/oder zweiten Futterfraktion ermittelt und ein Sensorwert an eine Steuerungselektronik zur Steuerung und/oder Regelung der Mischungsverhältnisse der Zutaten in den ersten und/oder zweiten Futterfraktionen und/oder der in den Zuchtbehälter einzufüllenden Mengen der ersten und/oder zweiten Futterfraktionen übermittelt. Über die Verwendung des Feuchtigkeitssensors kann der Feuchtegehalt des in einen Zuchtbehälter eingegebenen Futterbreis genau überwacht und dokumentiert werden. Es ist vorteilhaft, wenn der Feuchtegehalt mit dem Feuchtigkeitssensor auf einer Verfahrensstufe ermittelt wird, die vor der Abgabe einer Futterfraktion an den Zuchtbehälter liegt, wie beispielsweise in einem Mischer, so dass der Feuchtegehalt gegebenenfalls noch durch Zugabe von Wasser oder Trockenfutterkomponenten korrigiert werden kann, bevor die Abgabe in den Zuchtbehälter erfolgt.

Nach einer Ausgestaltung der Erfindung wird mit einem Leistungsaufnahmesensor die Rührleistung in einem Rührwerk für die Zubereitung der ersten und/oder zweiten Futterfraktion ermittelt und ein Sensorwert an eine Steuerungselektronik zur Steuerung und/oder Regelung der Mischungsverhältnisse der Zutaten in den ersten und/oder zweiten Futterfraktionen und/oder der in den Zuchtbehälter einzufüllenden Mengen der ersten und/oder zweiten Futterfraktionen übermittelt. Je nach Feuchtegehalt und Menge der zu rührenden Futterportion ist die Leistungsaufnahme des Rührwerks unterschiedlich. Die aktuelle Leistungsaufnahme des Motors, mit dem eine Futterportion im Rührwerk gerührt wird, ist somit ein guter Indikator für den Feuchtegehalt der im Rührwerk befindlichen Futterportion. Der Leistungsaufnahmesensor kann in ein automatisiertes Verfahren zur Herstellung und Abgabe einer Futterportion eingebunden sein.

Weitere Merkmale der Erfindung ergeben sich aus den Ansprüchen, den Figuren und der Figurenbeschreibung. Alle vorstehend in der Beschreibung genannten Merkmale und Merkmalskombinationen sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder aber in Alleinstellung verwendbar.

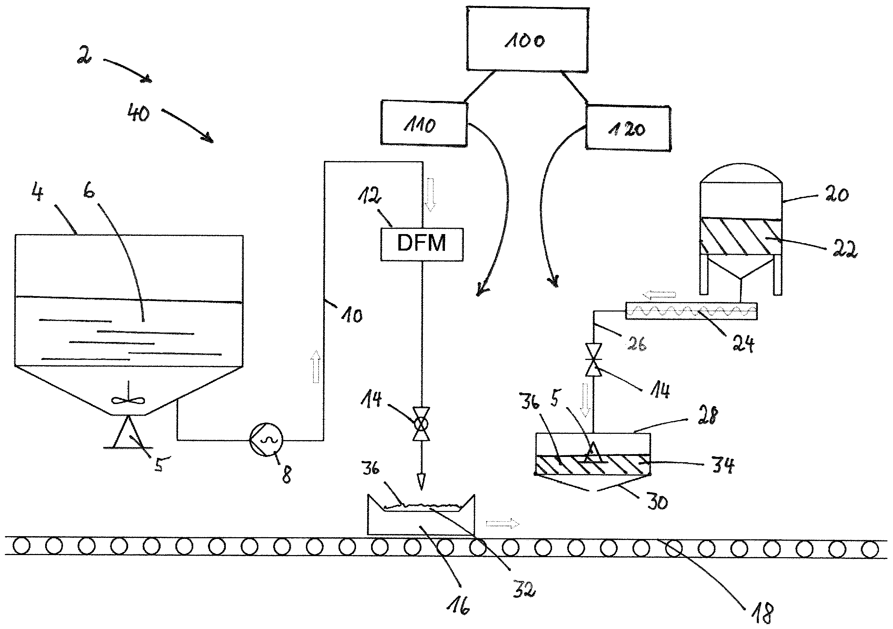

Nachfolgend werden beispielhaft Ausführungsbeispiele anhand eines Ausführungsbeispiels in der beiliegenden Fig. 1 beschrieben.

In der beigefügten Figur 1 ist als Ausschnitt aus einer Anlage 2 die Vorrichtung 40 zur Einbringung eines Futterbreis in einen Zuchtbehälter 16 gezeigt, die einen Mischbehälter 4 zur Bevorratung eines Flüssigfutters 6 aufweist. Der Mischbehälter 4 ist über eine Wiegevorrichtung 5 gewogen. Durch einen Vergleich der von der Wiegevorrichtung gewogenen Gewichte vor, während und am Ende eines Befüllvorgangs des Mischbehälters 4 kann die Menge einer in den Mischbehälter 4 eingefüllten Portion eines Flüssigfutters 6 ermittelt werden. Das in dem Mischbehälter 4 bevorratete Flüssigfutter 6 wird mit einer Pumpe 8 durch eine Rohrleitung als Fördermittel 10 bis zu einem Ventil 14 gepumpt, von dem aus das Flüssigfutter 6 bei einem geöffneten Ventil 14 in den Zuchtbehälter 16 läuft. Die Menge des vom Fördermittel 10 beförderten Flüssigfutters 6 kann von einem Durchflussmengenmesser 12 erfasst werden. Der Durchflussmengenmesser 12 kann mit einer elektronischen Steuerung verbunden sein, über die das Ventil 14 betätigt wird. Der Zuchtbehälter 16 befindet sich auf einem Förderband 18, mit dem der Zuchtbehälter 16 in die Richtung des Pfeils durch die Anlage 2 befördert werden kann. In den Zuchtbehälter 16 ist bereits eine Portion 36 des Flüssigfutters 6 als eine erste Futterfraktion 32 eingebracht worden.

Die Vorrichtung 40 weist auch einen Mischbehälter 20 zur Bevorratung eines Trockenfutters 22 auf. Bei dem Mischbehälter 20 kann es sich auch wie bereits vorstehend erläutert um ein Futtersilo oder sonstigen Behälter handeln, in dem sich eine einzelne Futterkomponente oder eine Mischung aus mehreren Futterkomponenten befindet. So können in einem oder mehreren einzelnen Behältern Weizenkleie, Körner von anderen Getreidearten, Orangenschalen, eine Vitamin- oder Mineralienmischung oder dergleichen befinden. Natürlich kann der Mischbehälter 20 auch dazu genutzt werden, eine Charge eines Trockenfutters 22 aus Futterkomponenten zusammen zu mischen, die dem Mischbehälter 20 zugeführt werden. Das Trockenfutter 22 wird mittels einer Förderschnecke 24, die in einer Rohrleitung als Fördermittel 26 angeordnet ist, in Richtung des Ventils 14 befördert. Bei geöffnetem Ventil 14 fällt das Trockenfutter 22 in den Klappkasten 28. Wenn sich die Klappen 30 des Klappkastens 28 öffnen, fällt die darin vorgehaltene Portion 36 der zweiten Futterfraktion 34 nach unten in den Zuchtbehälter 16, wenn dieser mit dem Förderband 18 zuvor in eine entsprechende Position bewegt worden ist. Das Trockenfutter 22 fällt dabei in das Flüssigfutter 6 der ersten Futterfraktion 32. Im Zuchtbehälter 16 können sich dann die beiden Futterfraktionen 32,34 miteinander vermischen. Im gezeigten Ausführungsbeispiel befindet sich der Klappkasten 28 an einer von der Abgabestelle des Flüssigfutters 6 in Förderrichtung abwärts gelegenen Position. Im Ausführungsbeispiel ist der Klappenkasten 28 verwogen, um Portionsgrößen möglichst genau zu ermitteln. Anders als bei einer gewichtsgesteuerten Zudosierung des Trockenfutters kann die Zudosierung mengenmäßig gesteuert auch über eine Zellenradschleuse, eine Förderschnecke, ein Förderband oder volumenmäßig gesteuert über einen Volumendosierer oder einer vergleichbaren Technik erfolgen.

Aus der Figur 1 ist ersichtlich, dass sich das Verfahren 100 in einen ersten Teilschritt 110, ein erster Anteil des gesamten in den Zuchtbehälter 16 einzubringenden Futterbreis mit einem höheren Flüssigkeitsanteil als erste Futterfraktion 32 und im zweiten Teilschritt 120 ein zweiter Anteil des gesamten in den Zuchtbehälter 16 einzubringenden Futterbreis mit einem niedrigeren Flüssigkeitsanteil als zweite Futterfraktion 34 in den Zuchtbehälter 16 eingebracht werden.

Das vorstehend beschriebene Ausführungsbeispiel dient nur der Erläuterung der Erfindung. Das Ausführungsbeispiel kann auf eine dem Fachmann als sachdienlich erscheinende Weise abgeändert werden, um sie an einen konkreten Anwendungsfall anzupassen.

### Bezugsziffernliste

- 2: Anlage
- 4: Mischbehälter
- 5: Wiegevorrichtung
- 6: Flüssigfutter
- 8: Pumpe
- 10: Fördermittel
- 12: Durchflussmengenmesser
- 14: Ventil
- 16: Zuchtbehälter
- 18: Förderband
- 20: Mischbehälter
- 22: Trockenfutter
- 24: Förderschnecke
- 26: Fördermittel
- 28: Klappkasten
- 30: Klappe
- 32: erste Futterfraktion
- 34: zweite Futterfraktion
- 36: Portion
- 40: Vorrichtung zur Einbringung eines Futterbreis
- 100: Verfahren
- 110: erster Teilschritt
- 120: zweiter Teilschritt

## Patentansprüche

1. Verfahren zur Bereitstellung von Futtermitteln in einer Anlage (2) zur Aufzucht von Insektenlarven, wobei in einer Zuchtphase ein Futterbrei und Insektenlarven in einen Zuchtbehälter (16) eingebracht werden, der Zuchtbehälter (16) über einen Zeitraum in einer Wärmekammer gelagert wird und am Ende des Zeitraums der Zuchtbehälter (16) aus der Wärmekammer entnommen und die Insektenlarven von verbliebenen Futterresten getrennt werden, **dadurch gekennzeichnet, dass** der Verfahrensschritt (100) des Einbringens des Futterbreis in den Zuchtbehälter (16) aufgeteilt wird in zumindest zwei Teilschritte (110, 120), wobei im ersten Teilschritt (110) ein erster Anteil des gesamten in den Zuchtbehälter (16) einzubringenden Futterbreis mit einem höheren Flüssigkeitsanteil als erste Futterfraktion (32) und im zweiten Teilschritt (120) ein zweiter Anteil des gesamten in den Zuchtbehälter (16) einzubringenden Futterbreis mit einem niedrigeren Flüssigkeitsanteil als zweite Futterfraktion (34) in den Zuchtbehälter (16) eingebracht werden.

2. Verfahren nach Anspruch 1, **dadurch gekennzeichnet, dass** es sich bei der zweiten Futterfraktion (34) um ein Trockenfutter (22) oder eine Trockenfutterkomponente handelt.

3. Verfahren nach Anspruch 2, **dadurch gekennzeichnet, dass** das Trockenfutter (22) oder die Trockenfutterkomponente vor der Einbringung in den Zuchtbehälter (16) in einem Mischbehälter (20) aus verschiedenen Futterkomponenten zusammengemischt wird, wobei die verwendeten Futterkomponenten aus zu der Anlage (2) gehörigen Vorratsbehältern entnommen und dem Mischbehälter (20) zugefördert werden.

4. Verfahren nach einem der vorhergehenden Ansprüche 2 oder 3, **dadurch gekennzeichnet, dass** das Trockenfutter (22) oder die Trockenfutterkomponente dem jeweiligen Zuchtbehälter (16) aus einem Klappkasten (28), einer Vorrichtung zur Volumen- oder Zeitdosierung oder einer Zellenradschleuse oder einer anderen Verteil- und Zudosiervorrichtung zugeführt wird.

5. Verfahren nach einem der vorhergehenden Ansprüche 2 - 4, **dadurch gekennzeichnet, dass** eine Portion (36) eines Trockenfutters (22) oder der Trockenfutterkomponente vor der Zuführung in den Zuchtbehälter (16) verwogen oder die Abgabemenge mittels einer Vorrichtung zur Durchlaufmengenmessung mengendosiert wird.

6. Verfahren nach einem der vorhergehenden Ansprüche 2 - 4, **dadurch gekennzeichnet, dass** eine Portion eines Flüssigfutters vor der Zuführung in den Zuchtbehälter (16) verwogen oder die Abgabemenge mittels einer Vorrichtung zur Durchflussmengenmessung (12) mengendosiert wird.

7. Verfahren nach einem der vorhergehenden Ansprüche, **dadurch gekennzeichnet, dass** zuerst die erste Futterfraktion (32) und danach die zweite Futterfraktion (34) in den Zuchtbehälter (16) eingegeben werden.

8. Verfahren nach einem der vorhergehenden Ansprüche, **dadurch gekennzeichnet, dass** mit einem Feuchtigkeitssensor der Feuchteanteil in der ersten und/oder zweiten Futterfraktion (34) ermittelt und ein Sensorwert an eine Steuerungselektronik zur Steuerung und/oder Regelung der Mischungsverhältnisse der Zutaten in den ersten und/oder zweiten Futterfraktionen (32, 34) und/oder der in den Zuchtbehälter (16) einzufüllenden Mengen der ersten und/oder zweiten Futterfraktionen (32, 34) übermittelt wird.

9. Verfahren nach einem der vorhergehenden Ansprüche, **dadurch gekennzeichnet, dass** mit einem Leistungsaufnahmesensor die Rührleistung in einem Rührwerk für die Zubereitung der ersten und/oder zweiten Futterfraktion (34) ermittelt und ein Sensorwert an eine Steuerungselektronik zur Steuerung und/oder Regelung der Mischungsverhältnisse der Zutaten in den ersten und/oder zweiten Futterfraktionen (32, 34) und/oder der in den Zuchtbehälter (16) einzufüllenden Mengen der ersten und/oder zweiten Futterfraktionen (32, 34) übermittelt wird.

10. Anlage (2) zur Aufzucht von Insektenlarven in Zuchtbehältern (16) mit einer Vorrichtung (40) zur Einbringung eines Futterbreis in einen Zuchtbehälter (16), **dadurch gekennzeichnet, dass** zur Einbringung des Futterbreis in den Zuchtbehälter (16) ein Mischbehälter (4) zur Bereitstellung eines Flüssigfutters (6) als erster Futterfraktion (32), ein Mischbehälter (20) zur Bereitstellung eines Trockenfutters (22) oder einer Trockenfutterkomponente als zweiter Futterfraktion (32) und separate Fördermittel (10, 26) für das Flüssigfutter (6) und das Trockenfutter (22) oder der Trockenfutterkomponente vorhanden sind, mit denen diese einem Zuchtbehälter (16) zugeleitet werden.

11. Anlage nach Anspruch 10, **dadurch gekennzeichnet, dass** die Vorrichtung zur Einbringung eines Futterbreis dazu ausgestattet ist, die Verfahrensschritte gemäß den Ansprüchen 1 bis 9 auszuführen.

## Claims

1. Method for providing feed in an installation (2) for rearing insect larvae, wherein a feed mash and insect larvae are introduced into a rearing container (16) in a rearing phase, the rearing container (16) is stored in a heat chamber for a period of time and, at the end of the period, the rearing container (16) is removed from the heat chamber and the insect larvae are separated from remaining feed residues, **characterized in that** the method step (100) of introducing the feed mash into the rearing container (16) is divided into at least two substeps (110, 120), wherein, in the first substep (110), a first proportion of the whole feed mash to be introduced into the rearing container (16), with a higher liquid content as a first feed fraction (32), is introduced into the rearing container (16), and in the second substep (120), a second proportion of the whole feed mash to be introduced into the rearing container (16) with a lower liquid content is introduced into the rearing container (16) as a second feed fraction (34).

2. Method according to claim 1, **characterized in that** the second feed fraction (34) is dry feed (22) or a dry feed component.

3. Method according to claim 2, **characterized in that** the dry feed (22) or the dry feed component is mixed together from different feed components in a mixing container (20) before the introduction into the rearing container (16), wherein the feed components used are removed from storage containers which are part of the installation (2) and conveyed to the mixing container (20) .

4. Method according to either of the preceding claims 2 or 3, **characterized in that** the dry feed (22) or the dry feed component is supplied to the respective rearing container (16) from a hinged box (28), a device for volume or time metering or a rotary valve or another distribution and metered addition device.

5. Method according to one of the preceding claims 2 - 4, **characterized in that** a portion (36) of a dry feed (22) or the dry feed component is weighed before being supplied into the rearing container (16) or the discharged quantity is metered by means of a device for flow rate measurement.

6. Method according to one of the preceding claims 2 - 4, **characterized in that** a portion of a liquid feed is weighed before it is supplied into the rearing container (16) or the discharged quantity is metered by means of a device for flow rate measurement (12).

7. Method according to one of the preceding claims, **characterized in that** first the first feed fraction (32) and then the second feed fraction (34) are input into the rearing container (16).

8. Method according to one of the preceding claims, **characterized in that** the moisture content in the first and/or second feed fraction (34) is calculated by a moisture sensor and a sensor value is communicated to a control electronic system for the open-loop and/or closed-loop control of the mixing ratios of the additives in the first and/or second feed fractions (32, 34) and/or the quantities of the first and/or second feed fractions (32, 34) to be poured into the rearing container (16).

9. Method according to one of the preceding claims, **characterized in that** the stirring performance in a stirring unit for preparing the first and/or second feed fraction (34) is calculated by a power consumption sensor and a sensor value is communicated to a control electronic system for the open-loop and/or closed-loop control of the mixing ratios of the additives in the first and/or second feed fractions (32, 34) and/or the quantities of the first and/or second feed fractions (32, 34) to be poured into the rearing container (16).

10. Installation (2) for rearing insect larvae in rearing containers (16) with a device (40) for introducing a feed mash into a rearing container (16), **characterized in that**, in order to introduce the feed mash into the rearing container (16), a mixing container (4) for providing a liquid feed (6) as the first feed fraction (32), a mixing container (20) for providing a dry feed (22) or a dry feed component as the second feed fraction (32), and separate conveying means (10, 26) for the liquid feed (6) and the dry feed (22) or the dry feed component are present, by means of which they are supplied to a rearing container (16).

11. Installation (2) according to claim 10, **characterized in that** the device for introducing a feed mash is equipped to perform the method steps according to claims 1 to 9.

## Revendications

1. Procédé permettant de fournir des aliments pour animaux dans une installation (2) d'élevage de larves d'insectes, dans lequel, dans une phase d'élevage, une pâtée alimentaire et des larves d'insectes sont introduites dans une boîte d'élevage (16), la boîte d'élevage (16) est stockée pendant une certaine période de temps dans une armoire chauffante, et à la fin de la période de temps, la boîte d'élevage (16) est retirée de l'armoire chauffante, et les larves d'insectes sont séparées des résidus alimentaires,
**caractérisé en ce que** l'étape de procédé (100) de l'introduction de la pâtée alimentaire dans la boîte d'élevage (16) est divisée en au moins deux étapes partielles (110, 120), dans lequel, à la première étape partielle (110), une première part de l'ensemble de la pâtée alimentaire à introduire dans la boîte d'élevage (16) est introduite avec une plus grande part de liquide comme première fraction alimentaire (32), et à la deuxième étape partielle (120), une deuxième part de l'ensemble de la pâtée alimentaire à introduire dans la boîte d'élevage (16) est introduite avec une part de liquide inférieure comme deuxième fraction alimentaire (34) dans la boîte d'élevage (16).

2. Procédé selon la revendication 1, **caractérisé en ce que** la deuxième fraction alimentaire (34) est un aliment sec (22) ou un composant d'aliment sec.

3. Procédé selon la revendication 2, **caractérisé en ce qu'**avant l'introduction dans la boîte d'élevage (16), l'aliment sec (22) ou le composant d'aliment sec est mélangé dans un récipient mélangeur (20) à partir de différents composants alimentaires, selon lequel les composants alimentaires utilisés sont prélevés dans des réservoirs faisant partie de l'installation (2) et acheminés vers le récipient mélangeur (20).

4. Procédé selon l'une quelconque des revendications précédentes 2 ou 3, **caractérisé en ce que** l'aliment sec (22) ou le composant d'aliment sec est acheminé à la boîte d'élevage (16) respective à partir d'un distributeur à clapet (28), d'un dispositif de dosage volumétrique ou dans le temps ou d'une vanne rotative ou d'un autre dispositif de distribution ou de dosage additionnel.

5. Procédé selon l'une quelconque des revendications précédentes 2 à 4, **caractérisé en ce qu'**une partie (36) de l'aliment sec (22) ou du composant d'aliment sec est pesée avant d'être acheminée dans la boîte d'élevage (16) ou la quantité distribuée est dosée en quantité au moyen d'un dispositif de mesure de quantité de passage.

6. Procédé selon l'une quelconque des revendications précédentes 2 à 4, **caractérisé en ce qu'**une partie d'un aliment liquide est pesée avant d'être acheminée dans la boîte d'élevage (16) ou la quantité distribuée est dosée en quantité au moyen d'un dispositif de mesure de quantité de passage (12).

7. Procédé selon l'une quelconque des revendications précédentes, **caractérisé en ce que** d'abord la première fraction alimentaire (32) et ensuite la deuxième fraction alimentaire (34) sont introduites dans la boîte d'élevage (16).

8. Procédé selon l'une quelconque des revendications précédentes, **caractérisé en ce qu'**un capteur d'humidité détermine la teneur en humidité dans la première et/ou la deuxième fraction alimentaire (34), et une valeur de capteur est transmise à une électronique de commande pour commander et/ou réguler les rapports de mélange des ingrédients dans la première et/ou la deuxième fraction alimentaire (32, 34) et/ou des quantités à verser dans la boîte d'élevage (16) de la première et/ou de la deuxième fraction alimentaire (32, 34).

9. Procédé selon l'une quelconque des revendications précédentes, **caractérisé en ce qu'**un capteur de puissance absorbée détermine la puissance d'agitation dans un agitateur pour la préparation de la première et/ou de la deuxième fraction alimentaire (34), et une valeur de capteur est transmise à une électronique de commande pour commander et/ou réguler les rapports de mélange des ingrédients dans la première et/ou la deuxième fraction alimentaire (32, 34) et/ou des quantités à verser dans la boîte d'élevage (16) de la première et/ou de la deuxième fraction alimentaire (32, 34) .

10. Installation (2) d'élevage de larves d'insectes dans des boîtes d'élevage (16), comprenant un dispositif (40) permettant d'introduire une pâtée alimentaire dans une boîte d'élevage (16), **caractérisée en ce que** pour l'introduction de la pâtée alimentaire dans la boîte d'élevage (16), un récipient mélangeur (4) pour fournir un aliment liquide (6) comme première fraction alimentaire (32), un récipient mélangeur (20) pour fournir un aliment sec (22) ou un composant d'aliment sec comme deuxième fraction alimentaire (32), et des moyens de transport séparés (10, 26) pour l'aliment liquide (6) et l'aliment sec (22) ou le composant d'aliment sec qui permettent de les acheminer à une boîte d'élevage (16) sont prévus.

11. Installation selon la revendication 10, **caractérisée en ce que** le dispositif permettant d'introduire une pâtée alimentaire est équipé pour exécuter les étapes de procédé selon les revendications 1 à 9.
